# EUROPEAN PATENT APPLICATION

(11) **EP 1 270 026 A1**
(43) Date of publication of application: **02.01.2003**
(21) Application number: 01305452.3
(22) Date of filing: 22.06.2001
(51) Int. Cl.: A61L 31/16, A61L 31/10, A61K 31/59

(54) **Stents comprising vitamin D analogues for restenosis inhibition**

(71) Applicant: Andersen, Erik, 4000 Roskilde (DK)
(72) Inventor: Andersen, Erik, 4000 Roskilde (DK)
(74) Representative: Wilkinson, Stephen John

(57) **Abstract**

A stent has associated therewith a polymeric means for containing a vitamin D analogue. The means is capable of delivering a controlled release of a therapetucially-effective amount of the vitamin D analogue to the surface of a blood vessel in which the stent, and associated means, is located. The polymeric means may comprise a coating of a biocompatible polymeric material, containing the vitamin D analogue, on the surface of the stent. Alternatively, the polymeric means may comprise a sheath of polymeric material containing the vitamin D analogue which encompasses at least a portion of the stent.

## Description

The present invention relates to stents. More particularly, it relates to a stent provided with means for delivering a therapeutically-effective dose of a vitamin D analogue.

This invention applies mainly to the field of the treatment of blood vessels exhibiting stenoses, and more generally to the field of the treatment of diseases of various anatomical ducts of the human or animal body such as, for example, the urinary ducts, especially the urethra, or else the digestive ducts, especially the oesophagus.

The technique of balloon angioplasty of arterial stenoses is well known. This technique comprises the introduction, into the vessel to be treated, of a probe carrying an expandable balloon. At the region of restricted blood flow in the vessel being treated the expandable balloon is expanded, to force back the walls of the vessel, so that blood flow in the region of the vessel being treated is re-established.

Unfortunately, in many cases where balloon angioplasty has been carried out, the region of the vessel originally treated can suffer from further blockage, known as restenosis, only a few months after the balloon expansion of the vessel. This further blockage is a result of injury caused to the vessel walls by the expansion of the balloon and is caused mainly by the proliferation of smooth muscle cells at the site of the injury.

The introduction, into the vessel, of a stent has the effect of preventing the immediate elastic return of the vessel walls after the balloon has been removed and, subsequently, of deterring the reoccurrence of the original constriction in the vessel. Unfortunately, the introduction of a stent does not prevent the proliferation of the smooth muscle cells at the treatment site and restenosis can still occur after the introduction of a stent into the vessel.

It is known that stents can be provided with coatings which have an anti-restenosis effect. Typically, a stent may be provided with a coating which is capable, when located in the vessel being treated, of releasing in a controlled manner a therapeutic amount of a biologically-active material which has an antiproliferative effect on the smooth muscle cells at the treatment site.

It is also known to provide, in association with a stent, a sheath which encompasses at least a portion of the stent and which sheath is formed of a polymeric material in which is incorporated a biologically-active material having an anti-proliferative effect on smooth muscle cells. In such devices, the polymeric material used to form the sheath may be such that the anti-proliferative material can diffuse from it to the walls of the vessel in which it is located. Alternatively, the polymeric material used to form the sheath or used as a coating on the sheath may be biodegradable such that over a period of time, as the biodegradable material biodegrades, the biologically-active material is released to the local environment of the vessel wall.

A.M. Kissmeyer and J.T. Mortensen, in XENOBIOTICA, 2000, Vol 30, No.8, 815-830, describe the pharmacokinetics and metabolism of vitamin D analogues in rats and minipigs. These compounds are believed, because of their reported antiproliferative effects, to be useful for the systemic treatment of various solid tumours. The present invention is based on the discovery that such compounds can be employed as controlled release agents that can be eluted from the surfaces of stents, particularly for the treatment of restenosis.

Accordingly, the present invention provides a stent and, associated therewith, a polymeric means containing a vitamin D analogue which means is capable of delivering a controlled release of a therapeutically-effective amount of vitamin D analogue to the surface of a vessel in which the stent and associated means is located.

The stent, with its associate means, can be used as a vascular stent, particularly as a cardiovascular stent. However, it also may be used to treat other vessels in the human or animal body where stents may be employed such as, for example, urinary ducts, especially the urethra, and the digestive ducts, especially the oesophagus.

In one embodiment the polymeric means associated with the stent is a coating of a suitable polymeric material on the surface of the stent. In another embodiment the polymeric means associated with the stent is a sheath of polymeric material encompassing at least a portion of the stent.

The vitamin D analogue used in the present invention will be a compound that has anti-proliferative activity against smooth muscle cells such that it has an anti-restenosis effect. The vitamin D analogue is preferably the compound Seocalcitol [1(S),3(R)-dihydroxy-20(R)-(5'-ethyl-5'-hydroxy-hepta-1'(E),3'(E)-dien-1'-yl)-9,10-secopregna-5(Z),7(E),10(19)-triene which is an analogue of 1α,25-dihydroxyvitamin D₃, the physiologically active form of vitamin D₃. The synthetic preparation of these vitamin D analogues is described by E. Binderup et al in Proceedings of the Eighth Workshop on Vitamin D, 5-10^{th} July, Paris, pp 192-193 and A.M. Kissmeyer et al, Biochemical Pharmacology, 53, 1997, 1087-1097.

Stents typically comprise a metal support formed from for example, stainless steel, tantalum, platinum, tungsten, gold, nickel-titanium alloy or platinum-indium alloy. It is also possible for the stent to comprise a non-metallic support such as one formed of a biologically-compatible polymeric material. Preferably, the stent is formed of a stainless steel support. The structures of, and manufacture of, stents are well known in the art. In particular, reference is made to WO 98/58600.

The stent, according to the present invention, is used in association with a polymeric means for containing a vitamin D analogue which means is capable of delivering, when located in the vessel being treated, a controlled release of a therapeutically-effective dose of the vitamin D analogue to the walls of the vessel.

In one embodiment, the stent is provided with a coating of a polymeric material which has the property of being biologically compatible and which is capable of binding to and/or retaining within its matrix the vitamin D analogue. The polymeric material will in the environment of the vessel, for instance the blood vessel, being treated be capable of releasing in a controlled manner a therapeutically-effective amount of the vitamin D analogue. Polymeric materials which may be used in this way include biodegradable polymers and non-biodegradable polymers, for example polycarboxylic acids, cellulosic polymers, gelatin, polyvinylpyrrolidone, maleic anhydride polymers, polyamides, polyvinyl alcohols, poly ethylene oxides, polypyrroles and polythiophenes. The polymeric material may be a hydrogel, preferably crosslinked, such as polyacrylic acid polymer hydrogels as disclosed in US-A-5,304,121.

Typically, as is known generally in the art of making stents, a stent may be provided with a coating of polymeric material containing the vitamin D analogue by dipping the stent into a solution of the polymeric material to form the coating and then dipping the coated stent into a solution of the vitamin D analogue. Alternatively, the stent may be dipped into a solution containing the polymeric material and the vitamin D analogue. Following the dipping procedure, the coated stent is dried to remove the solvent thus leaving, on the surface of the stent, a deposit of the polymeric material. As an alternative to providing the coating by dipping, the coating can be provided on to the surface of the stent by other processes, such as by spraying, rolling and by electrodeposition.

In another embodiment, the stent may be used in association with a sheath as described above. Such sheaths, as described in US-A-5,383,928, may be formed of a biodegradable polymeric material or of a non-biodegradable polymeric material. Reference to US-A-5,383,928 can be made to the types of degradable or non-degradable polymeric materials that may be used in the present invention. Typically, the sheath will be formed of an ethylene-vinyl acetate copolymer.

The present invention further provides a method of inhibiting cell growth in a vessel in a human or animal body which comprises inserting a stent according to the invention described herein into the vessel in the human or animal body.

## Claims

1. A stent having associated therewith a polymeric means containing a vitamin D analogue which means is capable of delivering a controlled release of a therapeutically-effective amount of vitamin D analogue to the surface of a vessel in which the stent and associated means is located.

2. A stent according to claim 1, wherein the polymeric means associated with the stent comprises a coating of a biocompatible polymeric material, containing the vitamin D analogue, on the surface of the stent.

3. A stent according to claim 1, wherein the polymeric means associated with the stent comprises a sheath of polymeric material containing the vitamin D analogue said sheath encompassing at least a portion of the stent.

4. A stent according to any one of claims 1 to 3, wherein the vitamin D analogue is Seocalcitol.

5. A stent according to any one of claims 1 to 4, wherein the vessel in which the stent is located is a blood vessel.

6. A method of inhibiting cell growth in a vessel in a human or animal body which comprises inserting a stent according to any one of claims 1 to 5 into the vessel in the human or animal body.
